# EUROPEAN PATENT APPLICATION

(11) **EP 4 616 847 A1**
(43) Date of publication of application: **17.09.2025**
(21) Application number: 23887993.6
(22) Date of filing: 07.11.2023
(51) Int. Cl.: A61K 9/06, A61K 31/485, A61K 31/55, A61P 11/02

(54) **NALTREXONE IN-SITU GEL NASAL SPRAY, PREPARATION METHOD THEREFOR, AND USE THEREOF**

(30) Priority: 07.11.2022 CN 202211382960
(71) Applicant: Shenzhen Sciencare Pharmaceutical Co., Ltd., Shenzhen, Guangdong 518000 (CN)
(72) Inventor: XIAO, Zhimei, Shenzhen, Guangdong 518000 (CN); ZHAO, Xiaoge, Zhuhai, Guangdong 518000 (CN); ZHANG, Tongtong, Zhuhai, Guangdong 518000 (CN); ZENG, Zhe, Shenzhen, Guangdong 518000 (CN); YIN, Shugui, Zhuhai, Guangdong 518000 (CN); QU, Wei, Zhuhai, Guangdong 518000 (CN); ZHANG, Tao, Shenzhen, Guangdong 518000 (CN); QIU, Xinmin, Shenzhen, Guangdong 518000 (CN)
(74) Representative: karo IP
(86) International application number: PCT/CN2023/130148
(87) International publication number: WO 2024/099304

(57) **Abstract**

Provided are a naltrexone in-situ gel nasal spray, a preparation method therefor, and use thereof. The naltrexone in-situ gel nasal spray includes the following components: a pharmaceutically acceptable salt of naltrexone, a nasal mucosa adhesive, a physical stabilizer, a chemical stabilizer, and a pH regulator, where the nasal mucosa adhesive includes one or more of poloxamer 407 and poloxamer 188; the physical stabilizer includes one or more of Tween 20, Tween 80, and hydroxypropyl-β-cyclodextrin. By means of the addition of the nasal mucosa adhesive, upon contact with the nasal mucosa, the nasal spray can be gelled at the nasal cavity temperature and attached to the nasal mucosa, so that the duration of drug action at an action site is prolonged, and the efficacy and the bioavailability are improved. Meanwhile, by means of the addition of the physical stabilizer, the viscosity of the nasal spray can be improved, and the stability of the nasal mucosa adhesive is maintained, such that the stability of the nasal spray is improved. Moreover, by means of the addition of the physical stabilizer, the osmotic pressure of the nasal spray can be adjusted, such that the compliance of a patient is improved.

## Description

### Cross-reference to Related Applications

This application claims the priority of Chinese patent application No. 202211382960.X, entitled "NALTREXONE IN-SITU GEL NASAL SPRAY, PREPARATION METHOD THEREFOR, AND USE THEREOF" and filed on November 27, 2022, the entirety of which is incorporated herein by reference.

### Field of the Invention

The present disclosure relates to the technical field of pharmaceutical preparations, and in particular, to a naltrexone in-situ gel nasal spray, a preparation method therefor, and use thereof.

### Background of the Invention

Allergic rhinitis (AR), also referred to as Tubingfei, is a common disease in otorhinolaryngology. It manifests as nasal symptoms caused by an IgE-mediated inflammatory response following exposure of atopic individuals to allergens. The primary symptoms include recurrent sneezing, clear nasal discharge, nasal congestion, and nasal pruritus. Patients are often accompanied by ocular pruritus, conjunctival congestion, and/or lacrimation.

The main treatment for allergic rhinitis is medication, which includes intranasal corticosteroids, antihistamines and leukotriene receptor antagonists. At present, most intranasal corticosteroid preparations on the market are liquid preparations such as nasal drops and sprays. These nasal preparations are convenient to use, but due to the movement of nasal mucocilia, the retention time of these preparations on the surface of nasal mucosa is short (15-30 min), and the bioavailability is low. Nasal ointment and nasal cream may prolong the retention time of the preparation in the nasal mucosa, but the viscosity thereof is high, which has shortcomings of inaccurate dosage, inconvenient use, and prone to discomfort for patients. In addition, long-term use of intranasal corticosteroids will cause certain side effects; common side effects are nasal itching, nasal allergies, and even nasal ulcers. Some patients may experience nasal bleeding, headaches, and eye pain. Therefore, there is a need for developing low-toxic and effective nasal preparations for the treatment of allergic rhinitis.

### Summary of the Invention

Based on this, it is necessary to provide a low-toxic and effective naltrexone in-situ gel nasal spray, a preparation method therefor and use thereof, improving the bioavailability of the drug and the patient's compliance.

In the first aspect, provided herein is a naltrexone in-situ gel nasal spray, including the following components:
a pharmaceutically acceptable salt of naltrexone, a nasal mucosa adhesive, a physical stabilizer, a chemical stabilizer and a pH regulator;
where the nasal mucosa adhesive includes one or more of poloxamer 407 and poloxamer 188; and
the physical stabilizer includes one or more of Tween 20, Tween 80, and hydroxypropyl-β-cyclodextrin.

In some embodiments, the pharmaceutically acceptable salt of naltrexone is present in an amount of 0.5-10 parts by weight, the nasal mucosa adhesive is present in an amount of 8-17 parts by weight, the physical stabilizer is present in an amount of 0.01 to 1 part by weight, and the chemical stabilizer is present in an amount of 0.01 to 1 part by weight;
preferably, the pharmaceutically acceptable salt of naltrexone is present in an amount of 0.5-5 parts by weight, the nasal mucosa adhesive is present in an amount of 8-17 parts by weight, the physical stabilizer is present in an amount of 0.01 to 0.5 part by weight, and the chemical stabilizer is present in an amount of 0.01 to 0.2 part by weight.

In some embodiments, the naltrexone in-situ gel nasal spray has a pH of 4.0-6.5; optionally 4.5-6.0.

In some embodiments, the pharmaceutically acceptable salt of naltrexone includes naltrexone hydrochloride.

In some embodiments, the chemical stabilizer includes one or more of EDTA-2Na, sodium bisulfite, sodium metabisulfite, and vitamin C;
the pH regulator includes one or more of hydrochloric acid, phosphoric acid, citric acid, acetic acid and lactic acid.

In some embodiments, a preservative is also included;
where the preservative meets at least one of the following conditions:
(1) based on an amount of the pharmaceutically acceptable salt of naltrexone, the preservative is present in an amount of 0.001 to 0.02 part by weight; optionally 0.01-0.02 part by weight;
(2) the preservative includes one or more of chlorobutanol and benzalkonium chloride.

In some embodiments, a solvent is also included;
optionally, the solvent includes water.

In the second aspect, provided herein is a preparation method of the naltrexone in-situ gel nasal spray, including the following steps:
mixing a pharmaceutically acceptable salt of naltrexone with partial solvent, to obtain a first solution;
adding a chemical stabilizer, a physical stabilizer and an optional preservative into the first solution, to obtain a second solution;
adding a nasal mucosa adhesive into the second solution, to obtain a third solution; standing the third solution at a first temperature to make it completely swell, adjusting a pH of the third solution with a pH regulator, and adding the remaining solvent, to obtain the naltrexone in-situ gel nasal spray;
where, the first temperature is 0-10°C; optionally 2-5°C.

In the third aspect, provided herein is use of the naltrexone in-situ gel nasal spray described in the first aspect in preparation of drugs for the treatment of allergic rhinitis.

In the fourth aspect, provided herein is a drug for the treatment of allergic rhinitis, including the naltrexone in-situ gel nasal spray described in the first aspect.

The naltrexone in-situ gel nasal spray, a preparation method therefor and use thereof are provided. By means of the addition of the nasal mucosa adhesive, upon contact with the nasal mucosa, the nasal spray can be gelled at the nasal cavity temperature and attached to the nasal mucosa, so that the duration of drug action at an action site is prolonged, and the efficacy and the bioavailability are improved. Meanwhile, by means of the addition of the physical stabilizer, the stability of the nasal mucosa adhesive is maintained, such that the stability of the nasal spray is improved. Moreover, by means of the addition of the physical stabilizer, an appropriate osmotic pressure of the nasal spray can be maintained, such that the sense of suffocation in the application of the drug can be reduced, and the compliance of a patient is improved.

### Brief Description of the Drawings

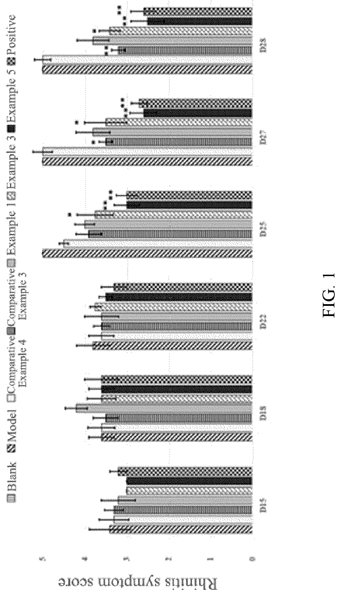

Fig. 1 shows a schematic diagram of the pharmacodynamic test results of Example 1, Example 3, Example 5, Comparative Example 3, Comparative Example 4, and commercially available preparation.

### Detailed Description of the Embodiments

In order to facilitate understanding of the present disclosure, a more comprehensive description of this disclosure is presented below with reference to the relevant drawings. Preferred embodiments of this disclosure are provided in the drawings. However, this disclosure may be implemented in many different forms, which is not limited to the embodiments described herein. Rather, these embodiments are provided for the purpose of providing a more thorough understanding of the disclosure.

Unless otherwise defined, all technical and scientific terms used herein have the same meaning as would normally be understood by a person skilled in the technical field of the present disclosure. The terms used herein in the specification are for the purpose of describing specific embodiments only and are not intended to limit the present disclosure.

**In** the present disclosure, the technical features described in terms of openness include a closed technical solution composed of the listed features and an open technical solution including the listed features.

**In** the present disclosure, a numerical range, unless otherwise specified, is regarded as continuous and includes the minimum and maximum values of the range and every value between such minimum and maximum values. Further, when a range refers to integers, it includes every integer between the minimum and maximum value of the range. In addition, when multiple ranges are provided to describe a feature or property, the ranges may be combined. In other words, unless otherwise specified, all ranges disclosed herein shall be understood to include any and all sub-ranges therein.

In the present disclosure, when dealing with the units of the data range, if there is "unit" only following the right endpoint, it means that the left endpoint and the right endpoint have the same unit. For example, "15-17 parts" means that the left endpoint "15" and the right endpoint "17" have the same unit of "part".

Only some numerical ranges are specifically disclosed in the present disclosure. However, any lower limit may be combined with any upper limit to form an undefined range; and any lower limit may be combined with any other lower limits to form an undefined range, just as any upper limit may be combined with any other upper limits to form an undefined range. In addition, each individually disclosed point or single value may itself be used as a lower or upper limit in combination with any other points or single values or in combination with other lower or upper limits to form an undefined range.

In the present disclosure, the temperature parameter, if not specially limited, allows both treatment at a constant temperature and treatment within a certain temperature range. The treatment at a constant temperature means that the temperature is allowed to fluctuate within the accuracy range controlled by the instrument.

In addition, the terms "first" and "second" are used for descriptive purposes only and are not to be understood as indicating or implying relative importance or as implicitly indicating the quantity of technical features indicated. In the description of the present disclosure, "more" means at least two, such as two, three, etc., unless otherwise expressly specified.

Unless otherwise specified, all embodiments and optional embodiments of the present disclosure may be combined to form a new technical solution. Unless otherwise specified, all technical features and optional technical features of the present disclosure may be combined to form a new technical solution.

Unless otherwise specified, all steps of the present disclosure may be carried out sequentially or randomly, preferably sequentially.

Provided herein is a naltrexone in-situ gel nasal spray, including the following components: a pharmaceutically acceptable salt of naltrexone, a nasal mucosa adhesive, a physical stabilizer, a chemical stabilizer and a pH regulator; where the nasal mucosa adhesive includes one or more of poloxamer 407 and poloxamer 188; the physical stabilizer includes one or more of Tween 20, Tween 80, and hydroxypropyl-β-cyclodextrin.

It should be noted that in-situ gels are a class of preparations that may undergo phase transition immediately at the site of administration after being administered in solution, transforming from liquid to non-chemically crosslinked semi-solid gels.

The naltrexone in-situ gel nasal spray mentioned above is a kind of in-situ gel that is sensitive to temperature change. When the ambient temperature is lower than the minimum critical phase transition temperature, it is in a liquid state, and when the ambient temperature is higher than the critical phase transition temperature, it is in a semi-solid gel state. The temperature of mammalian nasal cavity is about 32-35°C, and the critical phase transition temperature of the naltrexone in-situ gel nasal spray is 32-34°C.

It may be understood that by means of the addition of the nasal mucosa adhesive, upon contact with the nasal mucosa, the nasal spray can be gelled at the nasal cavity temperature, to form a three-dimensional network structure with hydrophilicity, which has good tissue compatibility and bioadhesion, and attached to the nasal mucosa, so that the duration of drug action at an action site is prolonged, and the efficacy and the bioavailability are improved. Meanwhile, by means of the addition of the physical stabilizer, the stability of the nasal mucosa adhesive is maintained, such that the stability of the nasal spray is improved. Moreover, by means of the addition of the physical stabilizer, an appropriate osmotic pressure of the nasal spray can be maintained, such that the sense of suffocation in the application of the drug can be reduced, and the compliance of a patient is improved.

The naltrexone in-situ gel nasal spray combines the advantages of liquid preparation and semi-solid preparation, makes up defects thereof. It can load drugs in liquid state, and a phase transition from liquid to semi-solid can occur at the site of action, which has unique advantages of tissue compatibility and sustained controlled release.

By means of the addition of the chemical stabilizer in the naltrexone in-situ gel nasal spray, an amount of related substances of the nasal spray can be reduced and stability of naltrexone is improved.

In some embodiments, the pharmaceutically acceptable salt of naltrexone is present in an amount of 0.5-10 parts by weight; for example, it may be 1-10 parts by weight, 1-9 parts by weight, 2-8 parts by weight, 3-7 parts by weight, 4-6 parts by weight, 4.5-5.5 parts by weight or 0.5-8 parts by weight. Further, the pharmaceutically acceptable salt of naltrexone is present in an amount of 0.5-5 parts by weight.

In some embodiments, the pharmaceutically acceptable salt of naltrexone include naltrexone hydrochloride. Allergic rhinitis belongs to a Type I hypersensitivity, since the body is stimulated by an allergen to produce antibodies, which bind to the surface of the nasal mucosa, as well as the cell membrane of mast cells and basophils. The technician of the present disclosure found through researches that the allergen can induce allergic rhinitis by activating TSLP/OX40L/OX40 signal and increasing the production of Th2 cytokines in a TLR4-dependent manner, therefore, TLR4 may play an important role in the pathogenesis of allergic rhinitis. Naltrexone can act as an opioid receptor antagonist on immune cells, indirectly regulate M1 cells, NK cells and other immune cells, and affect the secretion of various inflammatory cytokines, such as IL-2, IL-4, IL-12, IL-6 and TNF-α, thus affecting immune and inflammatory reactions. In addition, naltrexone can block the LPS-induced increase of TNF-α, NO and ROS by inhibiting the TLR4-TRIV-IRF3 signaling pathway. Therefore, naltrexone has the potential to treat allergic rhinitis by inhibiting TLR4 signaling pathway.

In some embodiments, based on 0.5-10 parts by weight of the pharmaceutically acceptable salt of naltrexone, the nasal mucosa adhesives is present in an amount of 8-17 parts by weight; for example, 9-17 parts by weight, 9-16 parts by weight, 10-15 parts by weight, 11-14 parts by weight, 12-13 parts by weight or 8-15 parts by weight, etc.

In some embodiments, based on 0.5-10 parts by weight of the pharmaceutically acceptable salt of naltrexone, the physical stabilizer is present in an amount of 0.01-1 part by weight; for example, 0.05-1 part by weight, 0.05-0.9 part by weight, 0.1-0.8 part by weight, 0.2-0.7 part by weight, 0.3-0.6 part by weight, 0.4-0.5 part by weight, or 0.01-0.7 part by weight, etc. Further, the physical stabilizer is present in an amount of 0.01-0.5 part by weight.

In some embodiments, based on 0.5-10 parts by weight of the pharmaceutically acceptable salt of naltrexone, the chemical stabilizer is present in an amount of 0.01-1 part by weight; for example, 0.05-1 part by weight, 0.05-0.9 part by weight, 0.1-0.8 part by weight, 0.2-0.7 part by weight, 0.3-0.6 part by weight, 0.4-0.5 part by weight, or 0.01-0.5 part by weight, etc. Further, the chemical stabilizer is present in an amount of 0.01-0.2 part by weight.

In some embodiments, the chemical stabilizer may include one or more of EDTA-2Na, sodium bisulfite, sodium metabisulfite, and vitamin C. Further, the chemical stabilizer is EDTA-2Na.

It should be noted that EDTA-2Na refers to ethylenediaminetetraacetic acid disodium salt.

In some embodiments, the naltrexone in-situ gel nasal spray has a pH of 4.0-6.5; for example, 4.5-6.5, 4.3-6.0, 4.5-5.7, 4.5-5.5, 5.0-5.5, 4.5-5.5 or 4.0-5.5, etc. Further, the naltrexone in-situ gel nasal spray has a pH of 4.5-6.0.

In some embodiments, the pH regulator may include one or more of hydrochloric acid, phosphoric acid, citric acid, acetic acid and lactic acid. Further, the pH regulator is selected from hydrochloric acid.

When hydrochloric acid is used as a pH regulator, the hydrochloric acid has a molar concentration of 0.01-0.5 mol/L; for example, 0.05-0.5 mol/L, 0.05-0.45 mol/L, 0.1-0.4 mol/L, 0.15-0.35 mol/L, 0.2-0.3 mol/L, 0.25-0.3 mol/L, 0.2-0.25 mol/L or 0.01-0.3 mol/L, etc. Further, the hydrochloric acid has a molar concentration of 0.05-0.2 mol/L.

In some embodiments, the naltrexone in-situ gel nasal spray also includes a preservative.

In some embodiments, based on 0.5-10 parts by weight of the pharmaceutically acceptable salt of naltrexone, the preservative is present in an amount of 0.001-0.02 part by weight; for example, 0.005-0.02 part by weight, 0.005-0.015 part by weight, 0.01-0.015 part by weight or 0.001-0.015 part by weight. Further, the preservative is present in an amount of 0.01-0.02 part by weight.

In some of these embodiments, the preservative may include one or more of chlorobutanol and benzalkonium chloride. Further, the preservative is selected from benzalkonium chloride.

In some embodiments, the naltrexone in-situ gel nasal spray also includes a solvent; optionally, the solvent includes water.

The present disclosure also provides a preparation method of the naltrexone in-situ gel nasal spray, including the following steps:
mixing a pharmaceutically acceptable salt of naltrexone with partial solvent, to obtain a first solution;
adding a chemical stabilizer, a physical stabilizer and an optional preservative into the first solution, to obtain a second solution;
adding a nasal mucosa adhesive into the second solution, to obtain a third solution; standing the third solution at a first temperature to make it completely swell, adjusting a pH of the third solution with a pH regulator, and adding the remaining solvent, to obtain the naltrexone in-situ gel nasal spray.

In the preparation process, it is conducive to improving the stability of the active pharmaceutical ingredient (API) and reducing the impurity content of the nasal spray by adding the chemical stabilizer followed the addition of the salt of naltrexone.

In some embodiments, the first temperature is 0-10°C; for example, 1-10°C, 1-9°C, 2-8°C, 3-7°C, 4-6°C, 4-5°C, 5-6°C, etc. Further, the first temperature is 2-5°C.

The present disclosure also provides use of the naltrexone in-situ gel nasal spray in preparation of drugs for the treatment of allergic rhinitis.

The present disclosure also provides a drug for the treatment of allergic rhinitis, including the naltrexone in-situ gel nasal spray.

The technical solution of the present disclosure is described in detail in combination with specific examples below.

### I. Preparation of the nasal spray

### 1. Formulation composition

The formulation compositions of Examples 1-10 and Comparative Examples 1-4 were shown in Table 1.

**Table 1**

| Compon ent | Main drug | | Nasal mucosa adhesive | | Preservative | | Chemical stabilizer | | Physical stabilizer | | pH regulator | | Solvent | | pH value |
|---|---|---|---|---|---|---|---|---|---|---|---|---|---|---|---|
| Main drug | Kind | Amount (mg) | Kind | Amou nt (mg) | Kind | Amo unt (mg) | Kind | Amo unt (mg) | Kind | Amo unt (mg) | Kind | Amo unt | Kind | Amo unt | |
| Example 1 | Naltrexo ne hydroch loride | 500 | Poloxa mer 407 | 8000 | Benzalk onium chloride | 20 | EDTA-2Na | 200 | Tween 20 | 200 | Dilute hydrochl oric acid | Prop er amou nt | Purifi ed water | Add to 100 mL | 4.5-6.0 |
| Example 2 | Naltrexo ne hydroch loride | 1000 | Poloxa mer 407 | 16500 | Benzalk onium chloride | 20 | EDTA-2Na | 100 | Tween 20 | 500 | Dilute hydrochl oric acid | Prop er amou nt | Purifi ed water | Add to 100 mL | 4.5-6.0 |
| Example 3 | Naltrexo ne hydroch loride | 2500 | Poloxa mer 407 | 16000 | Benzalk onium chloride | 15 | EDTA-2Na | 80 | Tween 20 | 100 | Dilute hydrochl oric acid | Prop er amou nt | Purifi ed water | Add to 100 mL | 4.5-6.0 |
| Example 4 | Naltrexo ne hydroch loride | 4000 | Poloxa mer 407 | 12500 | Benzalk onium chloride | 15 | EDTA-2Na | 50 | Tween 20 | 50 | Dilute hydrochl oric acid | Prop er amou nt | Purifi ed water | Add to 100 mL | 4.5-6.0 |
| Example 5 | Naltrexo ne hydroch loride | 5000 | Poloxa mer 407 | 17000 | Benzalk onium chloride | 10 | EDTA-2Na | 10 | Tween 20 | 10 | Dilute hydrochl oric acid | Prop er amou nt | Purifi ed water | Add to 100 mL | 4.5-6.0 |
| Example 6 | Naltrexo ne hydrochl oride | 500 | Poloxame r 407 | 8000 | Benzalk onium chloride | 20 | Sodium bisulfite | 100 | Tween 20 | 200 | Dilute hydroc hloric acid | Prop er amou nt | Purifi ed water | Add to 100 mL | 4.5-6.0 |
| Example 7 | Naltrexo ne hydrochl oride | 2500 | Poloxame r 407 | 16000 | Benzalk onium chloride | 15 | EDTA-2Na | 80 | Tween 80 | 100 | Dilute hydroc hloric acid | Prop er amou nt | Purifi ed water | Add to 100 mL | 4.5-6.0 |
| Example 8 | Naltrexo ne hydrochl oride | 5000 | Poloxame r 407 | 17000 | Benzalk onium chloride | 10 | EDTA-2Na | 10 | Tween 20 | 10 | Citric acid | Prop er amou nt | Purifi ed water | Add to 100 mL | 4.5-6.0 |
| Example 9 | Naltrexo ne hydrochl oride | 5000 | Poloxame r 407 | 17000 | Benzalk onium chloride | 10 | EDTA-2Na | 10 | Tween 20 | 10 | Lactic acid | Prop er amou nt | Purifi ed water | Add to 100 mL | 4.5-6.0 |
| Example 10 | Naltrexo ne hydrochl oride | 1000 | Poloxame r 407 | 16500 | Benzalk onium chloride | 20 | EDTA-2Na | 100 | Tween 20 | 500 | Dilute hydroc hloric acid | Prop er amou nt | Purifi ed water | Add to 100 mL | 4.5-6.0 |
| Comparati ve Example 1 | Naltrexo ne hydrochl oride | 1000 | Poloxame r 407 | 8000 | Benzalk onium chloride | 20 | / | / | Tween 20 | 200 | Dilute hydroc hloric acid | Prop er amou nt | Purifi ed water | Add to 100 mL | 4.5-6.0 |
| Comparati ve Example 2 | Naltrexo ne hydrochl oride | 2500 | Poloxame r 407 | 16000 | Benzalk onium chloride | 15 | EDTA-2Na | 80 | / | / | Dilute hydroc hloric acid | Prop er amou nt | Purifi ed water | Add to 100 mL | 4.5-6.0 |
| Comparative Example 3 | Naltrexo ne hydrochl oride | 2500 | Low methoxyl pectin | 125 | Benzalk onium chloride | 15 | EDTA-2Na | 80 | Tween 20 | 100 | Dilute hydroc hloric acid | Proper amount | Purified water | Add to 100 mL | 4.5-6.0 |
| Comparative Example 4 | / | / | Poloxamer 407 | 17000 | Benzalk onium chloride | 10 | EDTA-2Na | 10 | Tween 20 | 10 | Dilute hydroc hloric acid | Proper amount | Purified water | Add to 100 mL | 4.5-6.0 |

### 2. Preparation process

### Example 1

Naltrexone hydrochloride was added to 70% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 4°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 2

Naltrexone hydrochloride was added to 80% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 3°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 3

Naltrexone hydrochloride was added to 90% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 5°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 4

Naltrexone hydrochloride was added to 80% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 2°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 5

Naltrexone hydrochloride was added to 90% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 3°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 6

The preparation method of Example 6 was basically the same as that of Example 1, except that EDTA-2Na was replaced by sodium bisulfite, as follows:
Naltrexone hydrochloride was added to 70% of the prescribed amount of water, stirred and dissolved, to obtain a first solution;
sodium bisulfite, benzalkonium chloride and Tween 20 were added to the first solution, stirred and dissolved, to obtain a second solution;
poloxamer 407 was added to the second solution, then stood at 4°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 7

The preparation method of Example 7 was basically the same as that of Example 3, except that Tween 20 was replaced by Tween 80, as follows:
Naltrexone hydrochloride was added to 90% of the prescribed amount of water, stirred and dissolved, to obtain a first solution;
EDTA-2Na, benzalkonium chloride and Tween 80 were added to the first solution, stirred and dissolved, to obtain a second solution;
poloxamer 407 was added to the second solution, then stood at 5°C for 4 h to make the poloxamer 407 completely swell, dilute hydrochloric acid was added dropwise to adjust a pH value to 4.5-6.0, and water was added to replenish to 100 mL, to obtain the naltrexone in-situ gel nasal spray.

### Example 8

The preparation method of Example 8 was basically the same as that of Example 5, except that dilute hydrochloric acid was replaced by citric acid, as follows:
Naltrexone hydrochloride was added to 90% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 3°C for 4 h to make the poloxamer 407 completely swell, citric acid was added dropwise to adjust a pH value to 4.5-6.0, and water was added to replenish to 100 mL, to obtain the naltrexone in-situ gel nasal spray.

### Example 9

The preparation method of Example 9 was basically the same as that of Example 5, except that dilute hydrochloric acid was replaced by lactic acid, as follows:
Naltrexone hydrochloride was added to 90% of the prescribed amount of water, stirred and dissolved, to obtain a first solution;
EDTA-2Na, benzalkonium chloride and Tween 20 were added to the first solution, stirred and dissolved, to obtain a second solution;
poloxamer 407 was added to the second solution, then stood at 3°C for 4 h to make the poloxamer 407 completely swell, lactic acid was added dropwise to adjust a pH value to 4.5-6.0, and water was added to replenish to 100 mL, to obtain the naltrexone in-situ gel nasal spray.

### Example 10

The difference between the preparation method of Example 10 and that of Example 2 mainly lied in the addition sequence of poloxamer 407, as follows:
Naltrexone hydrochloride was added to 70% of the prescribed amount of water, stirred and dissolved, to obtain a first solution;
poloxamer 407 was added to the second solution, then stood at 4°C for 4 h to make the poloxamer 407 completely swell, to obtain a second solution;
EDTA-2Na, benzalkonium chloride and Tween 20 were added to the second solution, stirred and dissolved, then dilute hydrochloric acid was added dropwise to adjust a pH value to 4.5-6.0, and water was added to replenish to 100 mL, to obtain the naltrexone in-situ gel nasal spray.

### Comparative Example 1

The preparation method of Comparative Example 1 was basically the same as that of Example 1, except that EDTA-2Na was not added, as follows:
Naltrexone hydrochloride was added to 70% of the prescribed amount of water, stirred and dissolved, to obtain a first solution;
benzalkonium chloride and Tween 20 were added to the first solution, stirred and dissolved, to obtain a second solution;
poloxamer 407 was added to the second solution, then stood at 4°C for 4 h to make the poloxamer 407 completely swell, dilute hydrochloric acid was added dropwise to adjust a pH value to 4.5-6.0, and water was added to replenish to 100 mL, to obtain the nasal spray.

### Comparative Example 2

The preparation method of Comparative Example 2 was basically the same as that of Example 3, except that Twain 20 was not added, as follows:
Naltrexone hydrochloride was added to 90% of the prescribed amount of water, stirred and dissolved, to obtain a first solution;
EDTA-2Na and benzalkonium chloride were added to the first solution, stirred and dissolved, to obtain a second solution;
poloxamer 407 was added to the second solution, then stood at 5°C for 4 h to make the poloxamer 407 completely swell, dilute hydrochloric acid was added dropwise to adjust a pH value to 4.5-6.0, and water was added to replenish to 100 mL, to obtain the nasal spray.

### Comparative Example 3

The preparation method of Comparative Example 3 was basically the same as that of Example 3, except that poloxamer 407 was replaced by low methoxyl pectin, as follows:
Naltrexone hydrochloride was added to 90% of the prescribed amount of water, stirred and dissolved, to obtain a first solution;
low methoxyl pectin was added to the first solution, then stood at room temperature for 4 h to make the low methoxyl pectin completely swell, to obtain a second solution;
EDTA-2Na, benzalkonium chloride and Tween 20 were added to the second solution, stirred and dissolved; then dilute hydrochloric acid was added dropwise to adjust a pH value to 4.5-6.0, and water was added to replenish to 100 mL, to obtain the nasal spray.

### Comparative Example 4

The preparation method of Comparative Example 4 was basically the same as that of Example 5, except that naltrexone hydrochloride was not added, as follows:
Poloxamer 407 was added to 90% of the prescribed amount of water, then stood at 4°C for 4 h to make the poloxamer 407 completely swell, to obtain a first solution;
EDTA-2Na, benzalkonium chloride and Tween 20 were added to the first solution, stirred and dissolved, then dilute hydrochloric acid was added dropwise to adjust a pH value to 4.5-6.0, and water was added to replenish to 100 mL, to obtain the nasal spray.

### II. Performance test

### 1. Viscosity measurement

(1) Test sample: 10 mL of naltrexone in-situ gel nasal sprays prepared in Examples 1-10 and Comparative Examples 1-3 before subpackaging were filled into transparent glass vials, 30 bottles for each Example or Comparative Example respectively, and then capped and sealed as test samples.

### (2) Test method

The dynamic viscosity of the test sample was determined by a DVNEXT type rotational viscometer, with CPA-40Z spindle, a temperature of 25°C, a speed of 5.0 rpm (Refer to Method III of General Chapter 0633, Part IV of the 2020 Chinese Pharmacopoeia). Data within 10-90% of the measuring range of the instrument were read, the readings at 30s, 60s and 90s were recorded respectively, and an average value of three readings was recorded as the viscosity value of the test sample, the test sample having a viscosity value of 5-30 mPa·s was considered as meeting the use requirements.

(3) The test results of Example 3, Comparative Example 2 and Comparative Example 3 were shown in Table 2.

**Table 2**

| Group | Day 0 | Day 10, 40°C |
|---|---|---|
| | Viscosity (mP.s, speed: 5rpm) | Viscosity (mP.s, speed: 5rpm) |
| Example 1 | 7.82 | 7.90 |
| Example 2 | 28.64 | 28.44 |
| Example 3 | 27.45 | 27.14 |
| Example 4 | 20.09 | 19.88 |
| Example 5 | 29.10 | 28.91 |
| Example 6 | 7.46 | 7.38 |
| Example 7 | 27.30 | 27.09 |
| Example 8 | 28.94 | 28.85 |
| Example 9 | 28.83 | 28.77 |
| Example 10 | 26.97 | 27.14 |
| Comparative Example 1 | 7.51 | 7.43 |
| Comparative Example 2 | 25.92 | 11.14 |
| Comparative Example 3 | 4.25 | 3.95 |

As can be seen by comparing the results of Example 3 and Comparative Example 3 in Table 2, the system viscosity of the naltrexone in-situ gel nasal spray can be effectively improved by adding poloxamer 407, which is conducive to the retention of the drug at the administration site and the effect of the drug. Technicians found that when the amount of low methoxyl pectin was lower, the viscosity of the drug solution was very low, almost similar to the viscosity of water, and the local retention of the drug could not be increased; however, when the amount of low methoxyl pectin was higher, the drug solution becomes turbid with a yellowish milky appearance, and it was easy to grow microbe during storage.

As can be seen by comparing the results of Examples 3, 7 and Comparative Example 2 in Table 2, Tween 20 was conducive to maintaining the stability of the nasal mucosa adhesive, thereby improving the overall stability of the nasal spray. Technicians analyzed the reason and found it might be that Tween 20 was a condensation of Span and ethylene oxide, and had many hydrophilic polyoxyethylene groups in its molecule, so it had a strong hydrophilicity, which may enhance the dissolution of drugs and excipients, and then improve the stability of the system.

### 2. Osmotic pressure measurement

(1) Test sample: the naltrexone in-situ gel nasal sprays prepared in Example 1-10 and Comparative Example 1-3 before subpackaging.

(2) Test method: a freezing point osmometer (manufacturer: YASN, model: Osmo210) was used; the specific measuring method was as follows: 50 µL test sample was placed in a sample tube, the sample tube was placed in a sample chamber, then the instrument was clicked to start the measurement, and the osmotic pressure value of the test sample could be obtained at the end of the measurement. The test sample having an osmotic pressure of 280-380 mOsmol/kg was considered as meeting the use requirements.

(3) The test results were shown in Table 3.

**Table 3**

| Group | Exa mple 1 | Exa mple 2 | Exa mple 3 | Exa mple 4 | Exa mple 5 | Exa mple 6 | Exa mple 7 | Exa mple 8 | Exa mple 9 | Exa mple 10 | Com parat ive Exa mple 1 | Com parat ive Exa mple 2 | Com parat ive Exa mple 3 |
|---|---|---|---|---|---|---|---|---|---|---|---|---|---|
| Osmotic pressure value (mOsm ol/kg) | 289 | 340 | 330 | 305 | 362 | 290 | 550 | 369 | 370 | 357 | 286 | 331 | 280 |

As can be seen by comparing the results of Example 3 and Example 7 in Table 3, Tween 20 was conducive to maintaining the osmotic pressure of the nasal spray within a reasonable range and avoiding the discomfort of the patient caused by administration. When Tween 80 was used to replace Tween 20, the osmotic pressure of the nasal spray could not be maintained within a reasonable range, and the final osmotic pressure of the nasal spray was too high.

### 3. Stability test

(1) Test sample: 10 mL of naltrexone in-situ gel nasal sprays prepared in Examples 1-6, 8-10 and Comparative Examples 1 and 4 before subpackaging were filled into transparent glass vials, 30 vials for each Example or Comparative Example respectively, and then capped and sealed as test samples.

### (2) Test method

A. Measurement of naltrexone content: 1 mL of test sample was accurately measured, placed in a 100 mL volumetric flask, diluted with blank solvent to the mark, shaken well, 10 µL of the test sample was injected into a high-performance liquid chromatography, the peak area was recorded, and the drug concentration was calculated according to the standard curve equation.

### B. Measurement of relevant substance content

Preparation of test sample solution: 1 mL of test sample was accurately measured, placed in a 25 mL volumetric flask, diluted with blank solvent to the mark, shaken well, 10 µL of the test sample was injected into the high-performance liquid chromatography.

Preparation of control solution: 1 mL of test sample was accurately measured, placed in a 100 mL volumetric flask, diluted with blank solvent to the mark, shaken well, the 10 µL of the test sample was injected into the high-performance liquid chromatography.

If there was an impurity peak in the chromatogram of the test solution, the maximum impurity peak area should not exceed the main peak area of the control solution (0.5%), and the sum of the impurity peak area should not exceed 2.5 times the main peak area of the control solution (1.0%).

C. Appearance test: the test sample was visual observed, and the result was recorded.

(3) The test results were shown in Table 4.

**Table 4**

| Group | Day 0 | | | Day 10, 40°C | | | Day 30, 40°C | | |
|---|---|---|---|---|---|---|---|---|---|
| | Content (%) | Relevant substance (%) | Appearance | Content (%) | Relevant substance (%) | Appearance character | Content (%) | Relevant substance (%) | Appearance character |
| Example 1 | 99.9 | 0.099 | Colorless, clear solution | 99.8 | 0.198 | Colorless, clear solution | 99.6 | 0.352 | Colorless, clear solution |
| Example 2 | 99.9 | 0.081 | Colorless, clear solution | 99.7 | 0.271 | Colorless, clear solution | 99.4 | 0.550 | Colorless, clear solution |
| Example 3 | 99.9 | 0.118 | Colorless, clear solution | 99.9 | 0.138 | Colorless, clear solution | 99.6 | 0.322 | Colorless, clear solution |
| Examp le 4 | 99.9 | 0.151 | Colorless, clear solution | 99.9 | 0.132 | Colorless, clear solution | 99.7 | 0.298 | Colorless, clear solution |
| Examp le 5 | 99.9 | 0.080 | Colorless, clear solution | 99.7 | 0.251 | Colorless, clear solution | 99.5 | 0.463 | Colorless, clear solution |
| Example 6 | 92.8 | 7.103 | - | - | - | - | - | - | - |
| Example 8 | - | Interferen ce main peak | Colorless, clear solution | - | - | Colorless, clear solution | - | - | Colorless, clear solution |
| Example 9 | - | - | Yellow clear solution | - | - | - | - | - | - |
| Example 10 | 99.9 | 0.080 | Colorless, clear solution | 99.7 | 0.295 | Colorless, clear solution | 99.0 | 0.91 | Colorless, clear solution |
| Comparative Example 1 | 99.5 | 0.453 | Colorless, clear solution | 99.3 | 0.648 | Colorless, clear solution | 98.9 | 1.03 | Colorless, clear solution |
| Compa rative Example 4 | 0 | 0 | Colorless, clear solution | 0 | 0 | Colorless, clear solution | 0 | 0 | Colorless, clear solution |

| | | | | | | | | | |
|---|---|---|---|---|---|---|---|---|---|
| "-" indicated that the test item was far beyond the standard that may meet the use requirements, and the test was not performed. | | | | | | | | | |

As can be seen by comparing the results of Examples 1, 6 and Comparative Example 1 in Table 4, adding EDTA-2Na was conducive to maintaining the stability of naltrexone hydrochloride, showing that EDTA-2Na can prevent naltrexone hydrochloride from being oxidized by oxygen, which was conducive to improving the stability of the drug in the processes of preparation, storage and clinical use.

As can be seen by comparing the results of Examples 5, 8 and 9 in Table 4, hydrochloric acid as a pH regulator was more conducive to the stability of the formulation, with no interference for the main peak, and the appearance of the solution was normal. However, citric acid and lactic acid may react chemically with components in the system, produced new impurities that cause color changes and interference.

The main difference between Examples 2 and 10 was that poloxamer 407 was added in a different order during preparation. As can be seen by comparing the results of Example 2 and Example 10 in Table 4, after the test sample was placed for 30 days, a content of relevant substances in Example 10 was much higher than that in Example 2, and a content of relevant substances in Example 10 was close to 1%. The results showed that the order of feeding also affected the stability of the nasal spray preparation. After adding the main drug, the chemical stabilizer should be added as soon as possible, which was conducive to improving the stability of the preparation.

### III. Pharmacodynamic test

### 1. Test sample

In low dose group, medium dose group and high dose group of the test sample, the naltrexone in-situ gel nasal sprays prepared in Examples 1, 3 and 5 was administered respectively. In the common solution nasal spray group, the nasal spray prepared in Comparative Example 3 was administered. In the negative control group, the nasal spray prepared in Comparative Example 4 was administered. In the positive control group, triamcinolone acetonide nasal spray (purchased from Nanjing Xingyin Pharmaceutical Group Co., LTD., lot number 004210404) was administered. In the model control group and the blank control group, normal saline was administered.

### 2. Test method

(1) Animal species: Sprague-Dawley rat (SD rat); SPF-grade; 6-9 weeks of age; 190-280 g; female.

(2) Sensitization: On days D1, D3, D5, D7, D9, D11 and D13, animals in model control group, low dose group, medium dose group and high dose group of test samples, common solution nasal spray group, negative control group and positive control group were intraperitoneally injected with 1 mL sensitization solution (containing 0.3 mg ovalbumin + 30 mg aluminum hydroxide) for sensitization; animals in blank control group were intraperitoneally injected with 1 mL normal saline.

(3) Stimulation: From days D15 to D28, after animals were anesthetized with isoflurane, normal saline was instilled into the nasal cavities of animals in the blank control group, ovalbumin (20 mg/mL) was instilled into the nasal cavities of animals in the other groups. The dose was 10 µL/ side, once a day.

(4) Administration: On day D15, after stimulation of model animals, rhinitis symptoms were evaluated, and animals with a cumulative symptom score ≥3 points were administered with drugs on day D16. Animals in each group were administered with 10 µL drugs, that is, 5 µL for each side of the nasal cavity. If the cumulative score remained <3 points, animals were not administered with drugs temporarily, and stimulation and evaluation of allergy symptoms were continuously performed.

### (5) Evaluation of allergy symptoms

On days D16, D20 and D23, about 30 minutes of intranasal administration later, ovalbumin (100 mg/mL, 10 µL/ side) was administered intranasally, then animals were observed within 20 minutes for the nasal scratching times, sneezing times and severity of nasal discharge. The allergic rhinitis symptoms of animals were scored by using the cumulative scoring method (nasal scratching score + sneezing times score + nasal discharge). Rhinitis allergy symptoms were evaluated according to Table 5.

**Table 5**

| Index | Scoring standard | | | |
|---|---|---|---|---|
| | 0 point | 1 point | 2 points | 3 points |
| Nasal scratch | none | 1-4 | 5-10 | ≥11 |
| Sneeze | none | 1-3 | 4-9 | ≥10 |
| Nasal discharge | none | Inside the nostrils/one side | Full of nostrils | Full of snot on the face or massive nasal discharge |

### 3. Test results

The test results were shown in FIG. 1. After the sensitization stage, all groups exhibited cumulative symptom scores ≥3 points on days D15, indicating that the modeling was successful and the drug administration conditions were met. (1) At the stimulation stage, on days D15, D18 and D22, there were statistically significant differences between the model group and the blank control group, but there were no statistically significant difference between the treatment groups and the model group in behavioral scores; on days D25, D27 and D28, the score of the animals in the model group was 5; (2) The scores of common solution nasal spray group, low dose group, medium dose group, high dose group and positive control group were significantly decreased, with significant differences (P≤0.05, P≤0.01); (3) The rhinitis symptom scores of high dose group and positive control group were significantly decreased (P≤0.01), and there was no statistically significant difference between the high dose group and the positive control group; (4) The scores of the high dose group and the positive control group were lower and lower over time, and the results showed that the inhibition of allergic rhinitis of the high dose group was time-dependent.

As can be seen from the test results, common solution nasal spray, low dose, medium dose and high dose of the naltrexone in-situ gel nasal spray of the present disclosure can reduce the score of rhinitis inflammation, and medium dose and high dose of the naltrexone in-situ gel nasal spray can significantly reduce the score of rhinitis inflammation. As can be seen by comparing the scores of the common solution nasal spray group and the high dose group, the in-situ gel nasal spray was conducive to the local retention of the drug, so that the drug can play a better effect. Since the clinical symptoms of allergic rhinitis were runny nose, sneezing, nose itching, etc., the test results showed that medium and high doses of naltrexone in-situ gel nasal spray can inhibit allergic rhinitis, the inhibition of allergic rhinitis of the high dose was time-dependent, and the therapeutic effect of high dose was comparable to that of the positive control drug.

### IV. Chronic toxicity study

### 1. Test sample

The naltrexone in-situ gel nasal sprays prepared in Examples 1, 3 and 5 were used as the test samples in the low, medium and high dose groups respectively. Triamcinolone acetonide nasal spray (purchased from Nanjing Xingyin Pharmaceutical Group Co., LTD., lot number 004210404) was used in the positive control group.

### 2. Test method

(1) Administration: The rats were divided into 5 groups. In the blank control group, 5 µL of sodium chloride injection was instilled into each nasal cavity daily; in the experimental groups, 5 µL of test samples or positive control drug was instilled into each nasal cavity daily; and the experiment period was 2 weeks.

### (2) Histopathological examination

Gross observation: All animals in each group were necropsied and their tissues were preserved. After necropsy and tissue collection, animal carcasses were disposed of as medical waste.

During the necropsy, the nasal cavity of the animals was examined for erythema, edema, exudation, ulceration and other pathological alterations. The dorsal nasal skin was incised, the maxillary bone skin was removed, the maxillary bone was dissected free from the skull, the nasal cavity was cut along the nasal septum, the septal cartilage with mucosa were excised and rinsed with 2-8°C pre-chilled sodium chloride injection to eliminate blood and mucus, and put in 4% paraformaldehyde for fixation, decalcification, paraffin embedding, section, mounting, and HE staining. The nasal mucosa of animals was observed under microscope for histopathological examination such as mast cell and lymphoplasmacytic infiltration, glandular hyperplasia and capillary dilatation, as well as degree of squamous metaplasia of columnar epithelium.

### 3. Test results

(1) Body weight: The body weight of animals in each experimental group was basically the same in the first week of treatment. After 2 weeks, although not statistically significant, the trend of body weight gain and loss in the high dose group and positive control group was still significantly loss. **In** all experimental groups, compared with the blank control group, the body weight of animals in low dose group and medium dose group was similar to that of the normal group at the end of the experimental period, and the body weight of animals in the high dose group was slightly lower than that of the normal group, the low dose group and the medium dose group, but there was no statistical difference. The body weight of animals in the positive control group was significantly lower than that of normal group, and the difference was significant (P<0.05).

(2) Epithelial cell function: In the blank control group, the nasal epithelium, basement membrane, and lamina propria were normal, as expected, with pseudostratified ciliated columnar epithelium. The epithelium was characterized by a single layer of cells with nuclei at different locations and covered with cilia. In some animals, mild inflammatory infiltration in the epithelial cells was observed. Treatments with low dose, medium dose, and high dose did not cause changes in nasal epithelium, which were normal in all test samples and showed ciliary layers. All animals showed normal nasal epithelial cells, characterized by a single layer of goblet cells with nuclei distributed in different locations and covered by a layer of cilia.

In the positive control group, the nasal epithelium was undulated, the cell was vacuolated, the number of nuclei increased slightly, and some of them were hyperchromatic. In this group, the number of cilia in the epithelial cells was significantly decreased, and only one animal had normal mucosa. The majority of specimens in this group showed chronic mild inflammation and neutrophils were infiltrated from the lamina propria.

The results showed that test samples in three experimental groups had no effect on the body weight of rats, while the body weight of rats in the positive control group was significantly reduced. Compared with the blank control group, the nasal epithelium, basement membrane and lamina propria of rats in low dose group, medium dose group and high dose group had no significant changes. However, deep analysis of animal tissue sections treated in the positive control group showed that only one rat was observed showing normal nasal epithelium. **In** all other specimens, some changes were observed, such as ciliated cell reduction, cilia loss, goblet cell vacuolation, and moderate chronic inflammation. It may be concluded that the safety of the three dose groups was better than that of the positive control group.

The technical features in the above embodiments may be arbitrarily combined. To make the description concise, not all possible combinations of the technical features in the above-described embodiments are described. However, as long as there is no contradiction in the combination of these technical features, they should be considered to be within the scope of this specification.

The above-mentioned embodiments only express several implementation methods of the present invention, and the description is relatively specific and detailed, but it cannot be understood as limiting the scope of the invention patent. It should be pointed out that for ordinary technicians in this field, several modifications and improvements can be made without departing from the concept of the present invention, which all belong to the protection scope of the present invention.

## Claims

1. A naltrexone in-situ gel nasal spray, **characterized by** comprising the following components:
a pharmaceutically acceptable salt of naltrexone, a nasal mucosa adhesive, a physical stabilizer, a chemical stabilizer and a pH regulator;
wherein the nasal mucosa adhesive comprises one or more of poloxamer 407 and poloxamer 188; and
the physical stabilizer comprises one or more of Tween 20, Tween 80, and hydroxypropyl-β-cyclodextrin.

2. The naltrexone in-situ gel nasal spray according to claim 1, **characterized in that** the pharmaceutically acceptable salt of naltrexone is present in an amount of 0.5-10 parts by weight, the nasal mucosa adhesive is present in an amount of 8-17 parts by weight, the physical stabilizer is present in an amount of 0.01 to 1 part by weight, and the chemical stabilizer is present in an amount of 0.01 to 1 part by weight;
preferably, the pharmaceutically acceptable salt of naltrexone is present in an amount of 0.5-5 parts by weight, the nasal mucosa adhesive is present in an amount of 8-17 parts by weight, the physical stabilizer is present in an amount of 0.01 to 0.5 part by weight, and the chemical stabilizer is present in an amount of 0.01 to 0.2 part by weight.

3. The naltrexone in-situ gel nasal spray according to claim 1, **characterized in that** the naltrexone in-situ gel nasal spray has a pH of 4.0-6.5; optionally 4.5-6.0.

4. The naltrexone in-situ gel nasal spray according to claim 1, **characterized in that** the pharmaceutically acceptable salt of naltrexone comprises naltrexone hydrochloride.

5. The naltrexone in-situ gel nasal spray according to claim 1, **characterized in that** the chemical stabilizer comprises one or more of EDTA-2Na, sodium bisulfite, sodium metabisulfite, and vitamin C;
the pH regulator comprises one or more of hydrochloric acid, phosphoric acid, citric acid, acetic acid and lactic acid.

6. The naltrexone in-situ gel nasal spray according to any one of claims 1-5, **characterized by** further comprising a preservative;
wherein the preservative meets at least one of the following conditions:
(1) based on an amount of the pharmaceutically acceptable salt of naltrexone, the preservative is present in an amount of 0.001 to 0.02 part by weight; optionally 0.01-0.02 part by weight; and
(2) the preservative comprises one or more of chlorobutanol and benzalkonium chloride.

7. The naltrexone in-situ gel nasal spray according to any one of claims 1-5, **characterized by** further comprising a solvent;
optionally, the solvent comprising water.

8. A preparation method of the naltrexone in-situ gel nasal spray according to any one of claims 1-7, **characterized by** comprising the following steps:
mixing a pharmaceutically acceptable salt of naltrexone with partial solvent, to obtain a first solution;
adding a chemical stabilizer, a physical stabilizer and an optional preservative into the first solution, to obtain a second solution;
adding a nasal mucosa adhesive into the second solution, to obtain a third solution; standing the third solution at a first temperature to make it completely swell, adjusting a pH of the third solution with a pH regulator, and adding the remaining solvent, to obtain the naltrexone in-situ gel nasal spray;
wherein the first temperature is 0-10°C; optionally 2-5°C.

9. Use of the naltrexone in-situ gel nasal spray according to any one of claims 1-7 in preparation of drugs for the treatment of allergic rhinitis.

10. A drug for the treatment of allergic rhinitis, **characterized by** comprising the naltrexone in-situ gel nasal spray according to any one of claims 1-7.
